(19) Europäisches Patentamt
European Patent Office
Office européen des brevets

(11) Publication number: **0 364 548 B1**

(12) **EUROPEAN PATENT SPECIFICATION**

(45) Date of publication of patent specification: **09.06.93**

(51) Int. Cl.5: **C07C 69/75**, C09K 19/12, C09K 19/30, C07C 43/23, C07C 25/18, C07C 39/367

(21) Application number: **89904023.2**

(22) Date of filing: **25.03.89**

(86) International application number: **PCT/EP89/00336**

(87) International publication number: **WO 89/09203 (05.10.89 89/24)**

(54) **OCTAFLUOROBIPHENYLS.**

(30) Priority: **30.03.88 GB 8807572**

(43) Date of publication of application: **25.04.90 Bulletin 90/17**

(45) Publication of the grant of the patent: **09.06.93 Bulletin 93/23**

(84) Designated Contracting States: **CH DE FR GB IT LI NL**

(56) References cited:
**EP-A- 0 011 002**

**Journal of Organometallic Chemistry, volume 22, no. 2, 1970, Elsevier Sequoia S.A., (Lausanne, CH); W.I. RESPESS et al.: "Anomalous reactions of organomagnesium reagents with perfluoroaromatic compounds", pages 251 - 263**

(73) Proprietor: **MERCK PATENT GESELLSCHAFT MIT BESCHRÄNKTER HAFTUNG
Frankfurter Strasse 250 Postfach 4119
W-6100 Darmstadt(DE)**

(72) Inventor: **BYRON, David, John
29 Oak Tree Drive
Gelding Nottingham NH4 4DA(GB)**
Inventor: **BROWN, John, William
The Green
Fritchley Derbyshire DE5 2FW(GB)**
Inventor: **WILSON, Robert, Charles
Highlow Kirklington Road
Hockerton, Southwell Notts NG25 OPJ(GB)**
Inventor: **SAGE, Ian, Charles
58 Wentworth Drive
Broadstone Dorset BH18 8EG(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.6/3.3.1)

## Description

The invention relates to a liquid crystalline mixture with at least two liquid crystalline compounds characterized by the fact that at least one compound is a liquid crystalline compound of formula I

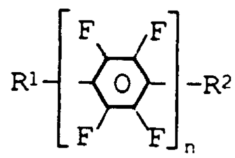

I

wherein n is 2, 3 or 4 and $R^1$ and $R^2$ each signify a group of formula

$R-(A^1-Z^1)_p-(A^2-Z^2)_q-$

in which

| | |
|---|---|
| R | is F, OH, Cl, Br, CN, NCO, NCS, $NO_2$, NC or alkyl or perfluoroalkyl wherein alkyl each has 1-15 C atoms, wherein one or two non-adjacent $CH_2$ or $CF_2$ groups, respectively, may be replaced by -O-, -CO-, -O-CO-, -CO-O-, -O-CO-O-, -C≡C- and/or -CH=CH-, |
| $A^1$ and $A^2$ | independently are an unsubstituted or mono- or polysubstituted 1,4-cyclohexylene group, wherein one or two non-adjacent $CH_2$ groups may be replaced by -O- and/or -S-, or denote a 1,4-bicyclo[2,2,2]octylene group, or a 1,4-phenylene group which is unsubstituted or substituted by one or up to four F and/or one or two Cl atoms and/or $CH_3$ groups and/or CN groups, wherein one or two CH groups may be replaced by N, |
| $Z^1$ | denotes -CO-O-, -O-CO-, $-CH_2CH_2-$, $-CHCN-CH_2-$, $-CH_2-CHCN-$, -CH=CH-, -C≡C-, $-OCH_2-$, $-CH_2O-$, -N=N-, -NO=N-, -N=NO- or a single bond, |
| $Z^2$ | denotes -CO-O-, -O-CO-, $-CH_2CH_2-$, $-CHCN-CH_2-$, $-CH_2-CHCN-$, CH=CH-, -C≡C-, $-OCH_2-$, $-CH_2O-$, -N=N-, -NO=N-, -N=NO-, $-(CH_2)_k-CO-O-$ or a single bond, |
| k | is 1 to 6, |
| p | is 0, 1 or 2, and |
| q | is 0 or 1, |

with the proviso that in the case n = 2

(a) the sum of p and q is 1, 2 or 3 and/or

(b) the sum of p and q is 0 and at least one of the rests R is alkyl or perfluoralkyl wherein alkyl hat 1-15 C atoms, wherein one or two non-adjacent $CH_2$ or $CF_2$ groups, respectively, may be replaced by -O-, -CO-, -O-CO-, -CO-O-, -O-CO-O-, -C≡C- and/or -CH=CH-and in particular means whereby liquid crystalline mixtures can be provided having physical properties optimised for practical applications.

Liquid crystal phases are commonly exhibited by organic compounds having extended rod-like molecules, and are characterised in their liquid crystalline state by a degree of order intermediate between those of a crystalline solid and of an isoptropic liquid respectively. The wide-spread use of liquid crystalline materials in electrooptic devices arises from their combination of fluid-like flow with an anisotropy of their physical properties that is typical for a crystalline material. When a liquid cystal material is utilised in an electro-optic device, optimal performance can only be obtained when the physical properties of the material are adjusted to extreme or optimal values to suit the particular application and device geometry in use. Examples of the physical properties which may beneficially be changed to improve the utility of a liquid crystalline material for a particular application include such properties as the mesogenic phase range, the dielectric constants, the elastic constants, the viscosity coefficient, and the refractive indices of the material.

The present invention provides a novel family of liquid crystal compounds which may be used by themselves or in admixture with other liquid crystal compounds classes to provide mixtures having advantageous combinations of liquid crystal properties, in particular low values of refractive indices and/or birefringence. It is well known that the performance of known optical and electro-optic devices can be improved by the reduction of the refractive indices or birefringence of the liquid crystalline material contained therein. For example, in the electrooptic display based upon the twisted nematic mode of operation, the off-state transmission of light only achieves its ideal value for discreet values of the parameter U where U is defined by the relation

$U = 2D\Delta n/\lambda$

where D is the thickness of the display cell, $\Delta n$ is the birefringence of the liquid crystal and $\lambda$ is the average wavelength of visible light and optimal performance in the display cell is obtained for values of U equal to $\sqrt{3}$, $\sqrt{15}$, etc. To allow use of a liquid crystalline material in a display cell it is therefore desirable that its birefringence should be adjusted so that the above equation is satisfied for the particular cell thickness which is chosen. In liquid crystal display cells containing dichroic-dyed liquid cystal materials and operating in the cholesteric to nematic phase-change mode otherwise known as the White-Taylor mode of operation, the birefringence of the liquid crystalline host mixture leads to the undesirable propagation of eliptically polarised light rays in the display cell which diminishes the optical efficiency of the display devices. It is therefore desirable when designing liquid crystal materials for use in this type of display, to adjust the birefringence of the liquid crystalline phase to the smallest practical value in order to obtain the best performance from the display. In electro-optic switching devices in which the liquid crystal material is used on an overlay on a planar optical waveguide, or as a cladding material on a fibre waveguide, it is essential that at least one of the refractive indices of the liquid crystalline material used is lower in value than that of the waveguide material or else the structure will no longer sustain the propagation of light within the waveguide.

According to the mode of operation chosen for such a device, it may be required to have the waveguide refractive index intermediate between the two refractive indices of the liquid crystalline overlayer, or to have the refractive index of the waveguide higher than either of the refractive indices of the liquid crystalline material. In liquid crystal mixtures intended for use in the NCAP display mode the clarity of the "ON" state of the device depends upon the accurate matching of the ordinary refractive index of the LC material to the refractive index of the supporting polymer matrix. The ability to alter the absolute refractive indices of the liquid crystal therefore both facilitates the formulation of mixtures for use in this device, and offers the opportunity to utilise a wider range of supporting polymers than would otherwise be possible.

For simplicity, in the following text $PheF_4$ is a tetrafluoro-1,4-phenylene group, Cy is a 1,4-cyclohexylene group, Dio is a 1,3-dioxane-2,5-diyl group, Bi is a bicyclo[2,2,2]octylene-1,4-diyl group, Phe is a 1,4-phenylene group, Pym is pyrimidine-2,5-diyl group and Pyr is a pyridine-2,5-diyl group, it being possible for Cy and/or Phe to be unsubstituted or substituted by one or up to four F and/or one or two Cl atoms and/or one or two $CH_3$ groups and/or one or two CN groups.

The birefringence and refractive indices of organic compounds and in particular those of liquid crystalline compounds are dependent on the electronic polarisability of the constituent molecules. Liquid crystal compounds having a largely aromatic character are commonly characterised by large values of the refractive indices and the birefringence. It has now been found unexpectedly that the compounds of formula I uniquely combine a moderately large birefringence with low absolute values of the refractive indices and further uniquely provide liquid crystalline compounds with wide nematic phase ranges having an ordinary refractive index lower than that of a fused silica wave guide at ordinary temperatures and wavelengths. The compounds can therefore be used by themselves or in combination with other classes of liquid crystalline compounds to provide mixtures in which the physical properties, particularly the refractive indices are optimised for electro-optic device applications.

The compounds of formula I can be used as components of liquid crystalline mixtures in particular for displays based on the twisted nematic and super twisted nematic cell, the effect of deformation of aligned phases or the NCAP effect, and for optical switching devices using a liquid crystal overlayer on a waveguiding substrate.

The invention was based on the object of discovering new stable partially fluorinated materials which are suitable as components of conventional liquid crystalline mixtures.

Liquid crystals derived from 4,4'-disubstituted octafluorobiphenyl are also known (D. Demus et al.; Flüssige Kristalle in Tabellen, VEB Deutscher Verlag für Grundstoffindustrie, Leipzig 1974 (Vol I) and 1984 (Vol II)), but the known materials are derivatives of Schiff bases which suffer from photochemical, hydrolytic and oxidative instability rendering them unsuitable for use in electro-optic devices. Further these compounds of the prior art show their liquid crystalline phase behaviour at very high temperature, which also makes them unsuitable for device applications.

W. L. Respess and C. Tamborski (J. Organometal. Chem., 22 (1970) 251-263) describe the preparation of similar 4,4'-disubstituted octafluorobiphenyles, but there is no hint to liquid crystalline mixtures containing such compounds.

The compounds of formula I provide liquid crystalline compounds having their liquid crystalline phase ranges at conveniently low temperature and the standard mixing techniques known to those skilled in the art may be applied to formulate mixtures which are liquid crystalline in the room temperature region and below.

The compounds of the formula I have a wide range of application. Depending on the choice of the substituents, these compounds can be used as the base materials from which liquid crystalline mixtures are composed up to 50 % of the total constitutents; however, it is also possible for compounds of the formula I to be added to liquid crystalline base materials of other classes of compounds, in order to influence the optical anisotropy of such a dielectric.

The compounds of the formula I are colourless in the pure state. They are vary stable towards chemicals, heat and light.

The invention thus relates to liquid crystalline mixtures with at least two liquid crystalline compounds characterized in that at least one compound is a compound of formula I. The invention furthermore relates to the use of compounds of formula I as components of liquid crystalline mixtures, and electro-optic display devices containing such mixtures.

A further aspect of this invention are the novel liquid crystalline compounds of formula I

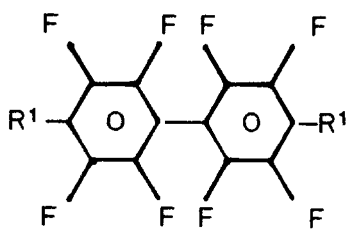

I

wherein $R^1$ and $R^2$ each signify a group of formula

$R\text{-}(A^1\text{-}Z^1)_p\text{-}(A^2\text{-}Z^2)_q\text{-}$

in which R, $Z^1$, $Z^2$, p and q have the meaning given, and

$A^1$ and $A^2$ independently are an unsubstituted or mono- or poly-substituted 1,4-cyclohexylene group, wherein one or two non-adjacent $CH_2$ groups may be replaced by -O- and/or -S-, or denote a 1,4-bicyclo[2,2,2]octylene group, or a 1,4-phenylene group,

with the proviso that the sum of p and q is 1, 2 or 3.

Above and below, $R^1$, $R^2$, R, n, p, q, k, $A^1$, $A^2$, $Z^1$ and $Z^2$ have the meaning given, unless expressly indicated otherwise.

The compounds of the formula I accordingly include preferred compounds with two rings of the part formula Ia:

$R\text{-}PheF_4\text{-}PheF_4\text{-}R$ Ia

compounds with three rings of part formulae Ib

$R\text{-}PheF_4\text{-}PheF_4\text{-}Z^2\text{-}A^2\text{-}R$ Ib

compounds with four rings of part formulae Ic and Id

$R\text{-}PheF_4\text{-}PheF_4\text{-}Z^1\text{-}A^1\text{-}Z^1\text{-}A^1\text{-}R$ Ic

$R\text{-}A^2\text{-}Z^2\text{-}PheF_4\text{-}PheF_4\text{-}Z^1\text{-}A^1\text{-}R$ Id

and compounds with five rings of part formulae Ie to If

$R\text{-}PheF_4\text{-}PheF_4\text{-}Z^2\text{-}A^2\text{-}(Z^1\text{-}A^1)_2\text{-}R$ Ie

$R\text{-}A^1\text{-}Z^1\text{-}PheF_4\text{-}PheF_4\text{-}Z^2\text{-}A^2\text{-}Z^1\text{-}A^1\text{-}R$ If

In the compounds of the formulae above and below, R preferably denotes independently F, alkyl, or furthermore alkoxy.

The groups $A^1$ and $A^2$ are preferably Cy, Phe, Dio, Pym, or Pyr; the compounds of the formula I preferably contain not more than one of the radicals Dio, Bi, Pym, or Pyr.

$Z^1$ and $Z^2$ are preferably single bonds, and secondly preferably -CO-O-, -O-CO- or -$CH_2CH_2$-groups. $Z^2$ may also preferably denote -$(CH_2)_k$-CO-O-, k is preferably 1 or 2. Particularly preferred are compounds wherein $Z^2$ denotes -CO-O-. Particularly preferred are compounds wherein $A^1$ and $A^2$ are Phe or Cy.

If R is an alkyl radical and/or alkoxy radical, this radical can be straight-chain or branched. Preferably, it is straight-chain and has 2, 3, 4, 5, 6 or 7 C atoms and is accordingly preferably ethyl, propyl, butyl, pentyl, hexyl, heptyl, ethoxy, propyloxy, butyloxy, pentyloxy, hexyloxy or heptyloxy, also methyl, octyl, nonyl, decyl, undecyl, dodecyl, tridecyl, tetradecyl, pentadecyl, methoxy, octyloxy, nonyloxy, decyloxy, undecyloxy, dodecyloxy, tridecyloxy or tetradecyloxy.

Oxaalkyl is preferably straight-chain 2-oxapropyl (= methoxymethyl), 2-(= ethoxymethyl) or 3-oxabutyl (= 2-methoxyethyl), 2-, 3- or 4-oxapentyl, 2-, 3-, 4- or 5-oxahexyl, 2-, 3-, 4-, 5- or 6-oxaheptyl, 2-, 3-, 4-, 5-, 6-, or 7-oxaoctyl, 2-, 3-, 4-, 5-, 6-, 7- or 8-oxanonyl or 2-, 3-, 4-, 5-, 6-, 7-, 8- or 9-oxadecyl.

If R is an alkyl radical in which a $CH_2$ group is replaced by -CH=CH-, it can be straight-chain or branched. Preferably, it is straight-chain and has 2 to 10 C atoms. It is accordingly, in particular, vinyl, prop-1- or prop-2-enyl, but-1-, -2- or -3-enyl, pent-1-, -2-, -3-or -4-enyl, hex-1-, -2-, -3-, -4- or -5-enyl, hept-1-, -2-, -3-, -4-, -5- or -6-enyl, oct-1-, -2-, -3-, -4-, -5-, -6- or -7-enyl, non-1-, -2-, -3-, -4-, -5-, -6-, -7- or -8-enyl or dec-1-, -2-, -3-, -4-, -5-, -6-, -7-, -8- or -9-enyl.

Compounds of the formula I with branched terminal groups R can occasionally be of importance because of an improved solubility in the customary liquid crystal base materials, but in particular as chiral doping substances if they are optically active.

Branched groups of this type as a rule contain not more than one chain branching. Preferred branched radicals R are isopropyl, 2-butyl (= 1-methylpropyl), isobutyl (= 2-methylpropyl), 2-methylbutyl, isopentyl (= 3-methylbutyl), 2-methylpentyl, 3-methylpentyl, 2-ethylhexyl, 2-propylpentyl, isopropoxy, 2-methylpropoxy, 2-methylbutoxy, 3-methylbutoxy, 2-methylpentoxy, 3-methylpentoxy, 2-ethylhexoxy, 1-methylhexoxy and 1-methylheptoxy.

Formula I includes both the racemates of these compounds and the optical antipodes, as well as mixtures thereof.

Those of the compounds of the formulae I, Ia to Ie in which at least one of the radicals contained therein has one of the preferred meanings mentioned are preferred.

Particular preferred compounds of the formula I are those of the part formulae I1 to I32:

R-$PheF_4$-$PheF_4$-R'    I1

R-$PheF_4$-$PheF_4$-Phe-R'    I2

R-$PheF_4$-$PheF_4$-Cyc-R'    I3

R-$PheF_4$-$PheF_4$-$PheF_4$-R'    I4

R-$PheF_4$-$PheF_4$-Z-Phe-R'    I5

R-$PheF_4$-$PheF_4$-Z-Cyc-R'    I6

R-$PheF_4$-$PheF_4$-Phe-Phe-R'    I7

R-Phe-$PheF_4$-$PheF_4$-Phe-R'    I8

R-Cyc-$PheF_4$-$PheF_4$-Phe-R'    I9

R-Cy-$PheF_4$-$PheF_4$-Cy-R'    I10

R-$PheF_4$-$PheF_4$-Cy-Cy-R'    I11

R-$PheF_4$-$PheF_4$-Phe-Cy-R'    I12

R-$PheF_4$-$PheF_4$-Cy-Phe-R'    I13

R-$PheF_4$-$PheF_4$-$Phe_4$-Cy-R' I14

R-$PheF_4$-$PheF_4$-$Phe_4$-Phe-R' I15

R-$PheF_4$-$PheF_4$-$PheF_4$-$PheF_4$-R' I16

R-$PheF_4$-$PheF_4$-Z-Phe-Phe-R' I17

R-$PheF_4$-$PheF_4$-Z-Cy-Phe-R' I18

R-$PheF_4$-$PheF_4$-Z-Phe-Cy-R' I19

R-$PheF_4$-$PheF_4$-Z-Cy-Cy-R' I20

R-$PheF_4$-$PheF_4$-Phe-Z-Phe-R' I21

R-$PheF_4$-$PheF_4$-Phe-Z-Cy-R' I22

R-Phe-Z-$PheF_4$-$PheF_4$-Phe-R' I23

R-Cy-Z-$PheF_4$-$PheF_4$-Phe-R' I24

R-Phe-Z-$PheF_4$-$PheF_4$-Cy-R' I25

R-Cy-Z-$PheF_4$-$PheF_4$-Cy-R' I26

R-$PheF_4$-$PheF_4$-$PHeF_4$-Z-Phe-R' I27

R-$PheF_4$-$PheF_4$-$PheF_4$-Z-Cy-R' I28

R-$PheF_4$-$PheF_4$-Z-$PheF_4$-$PheF_4$-R' I29

R-Phe-Z-$PheF_4$-$PheF_4$-Z-Phe-R' I30

R-Cy-Z-$PheF_4$-$PheF_4$-Z-Cy-R' I31

R-Cy-Z-$PheF_4$-$PheF_4$-Z-Phe-R' I32

In the preferred compounds of the part formulae I1 to I32 R and R' independently have one of the meanings of R in formula I. Preferably R is alkanoyloxy with up to 15 C atoms and R' denotes preferably alkyl with 2-15 C atoms, wherein one $CH_2$ group is replaced by -O-CO- or -CO-O. A particularly preferred meaning or R' is $-(CH_2)_r-O-CO-CH_2)_s-H$, wherein r is 1, 2, 3, 4, 5, 6, 7, 8, 9, 10, 11, 12 or 13 and s is 1, 2, 3, 4, 5, 6, 7, 8, 9, 10, 11, 12 or 13 (r + s) is 2 to 13.

In the compounds of the part formulae I1 to I32 the bridging groups Z denote each independently -CO-O-, -OCO-, $-CH_2-CH_2-$, -C≡C-, $-OCH_2-$ or $-CH_2O-$, preferably -CO-O-, -O-CO- or $-CH_2-CH_2-$. Particularly preferred are compounds wherein the $PheF_4$-groups are linked by an O-atom to the bridging group Z.

Particluarly preferred smaller groups of compounds of the formula I are those of formula II to XVI

II: alkyl-$PheF_4$-$PheF_4$-alkyl
alky)-$PheF_4$-$PheF_4$-alkoxy
alkyl-$PheF_4$-$PheF_4$-CO-O-alkyl
alkyl-$PheF_4$-$PheF_4$-O-CO-alkyl
alkoxy-$PHeF_4$-$PheF_4$-alkoxy

III: alkyl-$PheF_4$-$PheF_4$-A-alkyl
alkyl-$PheF_4$-$PheF_4$-A-alkoxy
alkoxy-$PheF_4$-$PheF_4$-A-alkoxy
alkoxy-$PheF_4$-$PheF_4$-A-alkyl
F-$PheF_4$-$PheF_4$-A-alkyl
F-$PheF_4$-$PheF_4$-A-alkoxy

IV: alkyl-$PheF_4$-$PheF_4$-CO-O-A-alkyl
alkyl-$PheF_4$-$PheF_4$-CO-O-A-alkoxy
alkoxy-$PheF_4$-$PheF_4$-CO-O-A-alkoxy
alkoxy-$PheF_4$-$PheF_4$-CO-O-A-alkyl
F-$PheF_4$-$PheF_4$-CO-O-A-alkyl
F-$PheF_4$-$PheF_4$-CO-O-A-alkoxy

V: alkyl-$PheF_4$-$PheF_4$-O-CO-A-alkyl
alkyl-$PheF_4$-$PheF_4$-O-CO-A-alkoxy
alkoxy-$PheF_4$-$PheF_4$-O-CO-A-alkoxy
alkoxy-$PheF_4$-$PheF_4$-O-CO-A-alkyl
F-$PheF_4$-$PheF_4$-O-CO-A-alkyl
F-$PheF_4$-$PheF_4$-O-CO-A-alkoxy
F-$PheF_4$-$PheF_4$-O-$CH_2$-A-alkyl
F-$PheF_4$-$PheF_4$-O-$CH_2$-A-alkoxy
alkoxy-$PheF_4$-$PheF_4$-O-$CH_2$-A-alkyl
alkoxy-$PheF_4$-$PheF_4$-O-$CH_2$-A-alkoxy
alkyl-$PheF_4$-$PheF_4$-O-$CH_2$-A-alkyl
alkyl-$PheF_4$-$PheF_4$-O-$CH_2$-A-alkoxy

VI: alkyl-$PheF_4$-$PheF_4$-A-A-alkyl
alkyl-$PheF_4$-$PheF_4$-A-A-alkoxy
alkoxy-$PheF_4$-$PheF_4$-A-A-alkyl
alkoxy-$PheF_4$-$PheF_4$-A-A-alkoxy
F-$PheF_4$-$PheF_4$-A-A-alkyl
F-$PheF_4$-$PheF_4$-A-A-alkoxy

VII: alkyl-A-$PheF_4$-$PheF_4$-A-alkyl
alkoxy-A-$PheF_4$-$PheF_4$-A-alkyl
alkoxy-A-$PheF_4$-$PheF_4$-A-alkoxy

VIII: alkyl-$PheF_4$-$PheF_4$-$PheF_4$-A-alkyl
alkyl-$PheF_4$-$PheF_4$-$PheF_4$-A-alkoxy
alkoxy-$PheF_4$-$PheF_4$-$PheF_4$-A-alkyl
alkoxy-$PheF_4$-$PheF_4$-$PheF_4$-A-alkoxy
F-$PheF_4$-$PheF_4$-$PheF_4$-A-alkyl
F-$PheF_4$-$PheF_4$-$PheF_4$-A-alkoxy

IX: alkyl-$PheF_4$-$PheF_4$-$PheF_4$-$PheF_4$-alkyl
alkoxy-$PheF_4$-$PheF_4$-$PheF_4$-$PheF_4$-alkyl
alkoxy-$PheF_4$-$PheF_4$-$PheF_4$-$PheF_4$-alkoxy
F-$PheF_4$-$PheF_4$-$PheF_4$-$PheF_4$-alkoxy
F-$PheF_4$-$PheF_4$-$PheF_4$-$PheF_4$-alkyl
F-$PheF_4$-$PheF_4$-$PheF_4$-$PheF_4$-F

X: alkyl-$PheF_4$-$PheF_4$-O-CO-A-A-alkyl
alkyl-$PheF_4$-$PheF_4$-O-CO-A-A-alkoxy
alkoxy-$PheF_4$-$PheF_4$-O-CO-A-A-alkyl
alkoxy-$PheF_4$-$PheF_4$-O-CO-A-A-alkoxy
F-$PheF_4$-$PheF_4$-O-CO-A-A-alkyl
F-$PheF_4$-$PheF_4$-O-CO-A-A-alkoxy

XI: alkyl-$PheF_4$-$PheF_4$-CO-O-A-A-alkyl
alkyl-$PheF_4$-$PheF_4$-CO-O-A-A-alkoxy
alkoxy-$PheF_4$-$PheF_4$-CO-O-A-A-alkyl
alkoxy-$PheF_4$-$PheF_4$-CO-O-A-A-alkoxy
F-$PheF_4$-$PheF_4$-CO-O-A-A-alkyl
F-$PheF_4$-$PheF_4$-CO-O-A-A-alkoxy

XII: alkyl-A-CO-O-$PheF_4$-$PheF_4$-CO-O-A-alkyl
alkyl-A-CO-O-$PheF_4$-$PheF_4$-CO-O-A-alkoxy
alkoxy-A-CO-O-$PheF_4$-$PheF_4$-CO-O-A-alkyl
alkoxy-A-CO-O-$PheF_4$-$PheF_4$-CO-O-A-alkoxy
alkoxy-A-O-CO-$PheF_4$-$PheF_4$-CO-O-A-alkyl
alkoxy-A-O-CO-$PheF_4$-$PheF_4$-CO-O-A-alkoxy
alkyl-A-O-CO-$PheF_4$-$PheF_4$-CO-O-A-alkyl

alkoxy-A-CO-O-$PheF_4$-$PheF_4$-O-CO-A-alkoxy
alkyl-A-CO-O-$PheF_4$-$PheF_4$-O-CO-A-alkoxy
alkyl-A-CO-O-$PheF_4$-$PheF_4$-O-CO-A-alkyl

XIII:
alkyl-$PheF_4$-$PheF_4$-$PheF_4$-CO-O-A-alkyl
alkyl-$PheF_4$-$PheF_4$-$PheF_4$-CO-O-A-alkoxy
alkoxy-$PheF_4$-$PheF_4$-$PheF_4$-CO-O-A-alkyl
alkoxy-$PheF_4$-$PheF_4$-$PheF_4$-CO-O-A-alkoxy
F-$PheF_4$-$PheF_4$-$PheF_4$-CO-O-A-alkyl
F-$PheF_4$-$PheF_4$-$PheF_4$-CO-O-A-alkoxy

XIV:
alkyl-$PheF_4$-$PheF_4$-$PheF_4$-O-CO-A-alkyl
alkyl-$PheF_4$-$PheF_4$-$PheF_4$-O-CO-A-alkoxy
alkoxy-$PheF_4$-$PheF_4$-$PheF_4$-O-CO-A-alkyl
alkoxy-$PheF_4$-$PheF_4$-$PheF_4$-O-CO-A-alkoxy
F-$PheF_4$-$PheF_4$-$PheF_4$-O-CO-A-alkyl
F-$PheF_4$-$PheF_4$-$PheF_4$-O-CO-A-alkoxy

XV:
alkyl-$PheF_4$-$PheF_4$-$PheF_4$-O-CO-$PheF_4$-alkyl
alkyl-$PheF_4$-$PheF_4$-$PheF_4$-O-CO-$PheF_4$-alkoxy
alkoxy-$PheF_4$-$PheF_4$-$PheF_4$-O-CO-$PheF_4$-alkyl
alkoxy-$PheF_4$-$PheF_4$-$PheF_4$-O-CO-$PheF_4$-alkoxy
F-$PheF_4$-$PheF_4$-$PheF_4$-O-CO-$PheF_4$-alkyl
F-$PheF_4$-$PheF_4$-$PheF_4$-O-CO-$PheF_4$-alkoxy
F-$PheF_4$-$PheF_4$-$PheF_4$-CO-O-$PheF_4$-alkyl
F-$PheF_4$-$PheF_4$-$PheF_4$-CO-O-$PheF_4$-alkoxy
alkoxy-$PheF_4$-$PheF_4$-$PheF_4$-CO-O-$PheF_4$-alkyl
alkoxy-$PheF_4$-$PheF_4$-$PheF_4$-CO-C-$PheF_4$-alkoxy
alkyl-$PheF_4$-$PheF_4$-$PheF_4$-CO-O-$PheF_4$-alkyl
alkyl-$PheF_4$-$PheF_4$-$PheF_4$-CO-C-$PheF_4$-alkoxy

XVI:
alkyl-$PheF_4$-$PheF_4$-CO-O-$PheF_4$-$PheF_4$-alkyl
alkyl-$PheF_4$-$PheF_4$-CO-O-$PheF_4$-$PheF_4$-alkoxy
alkoxy-$PheF_4$-$PheF_4$-CO-O-$PheF_4$-$PheF_4$-alkyl
alkoxy-$PheF_4$-$PheF_4$-CO-O-$PheF_4$-$PheF_4$-alkoxy
F-$PheF_4$-$PheF_4$-CO-O-$PheF_4$-$PheF_4$-alkyl
F-$PheF_4$-$PheF_4$-CO-O-$PheF_4$-$PheF_4$-alkoxy
F-$PheF_4$-$PheF_4$-O-CO-$PheF_4$-$PheF_4$-alkyl
F-$PheF_4$-$PheF_4$-O-CO-$PheF_4$-$PheF_4$-alkoxy
F-$PheF_4$-$PheF_4$-$CH_2$-O-$PheF_4$-$PheF_4$-alkyl
F-$PheF_4$-$PheF_4$-$CH_2$-O-$PheF_4$-$PheF_4$-alkoxy
F-$PheF_4$-$PheF_4$-O-$CH_2$-$PheF_4$-$PheF_4$-alkyl
F-$PheF_4$-$PheF_4$-O-$CH_2$-$PheF_4$-$PheF_4$-alkoxy
alkoxy-$PheF_4$-$PheF_4$-$CH_2$-O-$PheF_4$-$PheF_4$-alkyl
alkoxy-$PheF_4$-$PheF_4$-$CH_2$-O-$PheF_4$-$PheF_4$-alkoxy
alkyl-$PheF_4$-$PheF_4$$CH_2$-O-$PheF_4$-$PheF_4$-alkyl
alkyl-$PheF_4$-$PheF_4$-$CH_2$-O-$PheF_4$-$PheF_4$-alkoxy

In the preferred compounds of the groups II to XVI the rings A denote each independently a Phe and/or a Cy-group. The structure element A-A of the compounds of the subgroups VI, X and XI denotes Phe-Phe, Cy-Cy, Phe-Cy or Cy-Phe, preferably Phe-Phe or Cy-Phe.

In the compounds of the formula I, those stereoisomers in which the rings Cy are trans-1,4-disubstituted and/or Dio are trans-2,5-disubstituted are preferred. Those of the abovementioned formulae which contain one or more groups Dio, Pym and/or Pyr include in each case the two 2,5-position isomers.

In the compound of the formula I in which $A^1$ represents a Pym or Pyr ring which is substituted in the 2-position by R, R is preferably alkyl.

The compounds of the formula I are prepared by methods which are known per se, such as are described in the literature (for example in the standard works, such as Houben-Weyl, Methoden der Organischen Chemie [Methods of Organic Chemistry], Georg-Thieme-Verlag, Stuttgart), and in particular under reaction conditions which are known and suitable for the reactions mentioned in more detail here can also be used in this connection.

If desired, the starting substances can also be formed in situ, such that they are not isolated from the reaction mixture but are immediately reacted further to give the compounds of the formula I.

Esters of the formula I can be obtained by esterification of corresponding carboxylic acids (or their reactive derivatives) with alcohols or phenols (or their reactive derivatives).

The corresponding carboxylic acids and alcohols or phenols are known or can be prepared by processes analogous to known processes.

Particularly suitable reactive derivatives of the carboxylic acids mentioned are the acid halides, above all the chlorides and bromides, and furthermore the anhydrides, for example also mixed anhydrides, preferably those of the corresponding carboxylic acids and trifluoroacetic acid formed in situ by mixing these carboxylic acids with trifluoroacetic anhydride, azides or esters, in particular alkyl esters with 1-4 C atoms in the alkyl group.

Possible reactive derivatives of the alcohols or phenols mentioned are, in particular, the corresponding metal alcoholates or phenolates, preferably of an alkali metal, such as sodium or potassium.

The esterification is advantageously carried out in the presence of an inert solvent. Particularly suitable solvents are ethers, such as diethyl ether, di-n-butyl ether, THF, dioxane or anisole, ketones, such as acetone, butanone or cyclohexanone, amides, such as dimethylformamide or phosphoric acid hexamethyltriamide, hydrocarbons, such as benzene, toluene or xylene, halogenohydrocarbons, such as carbon tetrachloride or tetrachloroethylene, and sulfoxides, such as dimethylsulfoxide or sulfolane. Water-immiscible solvents can simultaneously be advantageously used for azeotropic distillation of the water formed during the esterification. An excess of an organic base, for example pyridine, quinoline or triethylamine, can occasionally also be used as the solvent for the esterification. The esterification can also be carried out in the absence of a solvent, for example by heating the components in the presence of sodium acetate. The reaction temperature is usually between -50° and +250°, preferable between -20° and +80°. At these temperatures, the esterification reactions have as a rule ended after 15 minutes to 48 hours.

In detail, the reaction conditions for the esterification depend largely on the nature of the starting substances used. Thus, a free carboxylic acid is as a rule reacted with a free alcohol or phenol in the presence of a strong acid, for example a mineral acid, such as hydrochloric acid or sulfuric acid. A preferred reaction procedure is the reaction of an acid anhydride or, in particular, an acid chloride with an alcohol, preferably in a basic medium, bases which are of importance being, in particular, alkali metal hydroxides, such as sodium hydroxide or potassium hydroxide, alkali metal carbonates or bicarbonates, such as sodium carbonate, sodium bicarbonate, potassium carbonate or potassium bicarbonate, alkali metal acetates, such as sodium acetate or potassium acetate, alkaline earth metal hydroxides, such as calcium hydroxide, or organic bases, such as triethylamine, pyridine, lutidine, collidine or quinoline. Another preferred embodiment of the esterification comprises first converting the alcohol or phenol into the sodium alcoholate or phenolate or potassium alcoholate or phenolate, for example by treatment with ethanolic sodium hydroxide solution or potassium hydroxide solution, isolating this product and suspending it in acetone or diethyl ether, together with sodium bicarbonate or potassium carbonate, with stirring, and adding a solution of the acid chloride or anhydride in diethyl ether, acetone or diemthylformamide to this suspension, advantageously at temperatures between about -25° and +20°.

The liquid crystalline mixtures according to the invention consist of 3 to 25, preferably 4 to 15, components, at least one of which is a compound of the formula I. The other constitutents are preferably chosen from nematic or nematogenic substances, in particular the known substances, from the classes of azoxybenzenes, benzylideneanilines, biphenyls, terphenyls, phenyl or cyclohexyl benzoates, phenyl or cyclohexyl cyclohexanecarboxylates, phenylcyclohexanes, cyclohexylbiphenyls, cyclohexylcyclohexanes, cyclohexylcyclohexenes, cyclhexylnaphthalenes, 1,4-bis-cyclohexylbenzenes, 4,4'-biscyclohexylbiphenyls, phenyl- or cyclohexylpyrimidines, phenylpyridines, phenyl-or cyclohexyldioxanes, phenyl- or cyclohexyldithianes, 1,2-bis-phenylethanes, 1,2-biscyclohexylethanes, 1-phenyl-2-cyclohexylethanes, optionally halogenated stilbenes, benzyl phenyl ethers, tolanes and substituted cinnamic acids.

The most important compounds which are possible constituents of such liquid crystalline mixtures can be characterized by the formula 1

$R^3$-L-G-E-$R^4$ 1

wherein L and E are each an unsubstituted or laterally fluoro- or cyano- substituted carbo- or hetero-cyclic ring system from the group comprising 1,4-disubstituted benzene and cyclohexane rings, 1,4-disubstituted 1-cyanocyclohexane rings, 4,4-disubstituted biphenyl, phenylcyclohexane and cyclohexylcyclohexane systems, 2,5-di-substituted naphthalene, di- and tetra-hydronaphthalene, quinazoline and tetrahydroquinazoline, G is

| | |
|---|---|
| -CH = CH- | -N(O) = N- |
| -CH = CY- | -CH = N(O)- |
| -C≡C- | $-CH_2-CH_2-$ |
| -CO-O- | $-CH_2-O-$ |
| -CO-S- | $-CH_2-S-$ |
| -CH = N- | -COO-Phe-COO- |

or a C-C single bond, Y is halogen, preferably chlorine, or -CN and $R^3$ and $R^4$ are alkyl, alkoxy, alkanoyloxy or alkoxycarbonyloxy with up to 18, preferably up to 8, carbon atoms, it also being possible for one $CH_2$ group non-adjacent to an oxygen atom to be replaced by -O-, -CH = CH- or -C≡C-, or that one of the radicals $R^3$ and $R^4$ may also denote CN, $NO_2$, $CF_3$, NCS, F, Cl or Br.

In most of these compounds, $R^3$ and $R^4$ are different from one another, one of these radicals usually being an alkyl or alkoxy group. However, other variants of the substituents envisaged can also be used. Many such substances or mixtures thereof are commercially available. All of these substances can be prepared by methods which are known from the literature.

The liquid crystalline mixtures according to the invention contain about 0.5 to 100, preferably 15 to 100 %, of one or more compounds of the formula I. Liquid crystalline mixtures which contains 25 - 100, particular 30 - 90 %, of one or more compounds of the formula I can be used advantageously in the mixtures according to the invention.

The liquid crystalline mixtures according to the invention are prepared in a manner which is customary per se. As a rule, the components are dissolved in one another, preferably at elevated temperature.

The liquid crystalline mixtures according to the invention can be modified by suitable additives such that they can be used in all the types of liquid crystal display elements disclosed to date.

Such additives are known to the expert and are described in detail in the literature. For example, it is possible to add conductive salts, preferably ethyldimethyldodecylammonium 4-hexyloxybenzoate, tetrabutylammonium tetraphenylboranate or complex salts of crown ethers (compare, for example, I. Haller et al., Mol.Cryst.Lig.Cryst. Volume 24, pages 249-258 (1973)) for improving the conductivity, dichoric dyestuffs for the production of coloured guest/host systems or substances for changing the dielectric anisotropy, the viscosity and/or the orientation of the nematic phases. Such substances are described, for example, in German Offenlegungsschrift 2,209,127, 2,240,863, 2,321,632, 2,338,281, 2,450,088, 2,637,430, 2,853,728 and 2,902,177.

The following examples are intended to illustrate the invention without limiting it. Percentages above and below are percentages by weight. All the temperatures are given in degrees Centrigrade. The symbols are furthermore as follows: Cr: crystlline solid state, S: smectic phase (the index characterizes the phase type), N: nematic phase, Ch: cholesteric phase, I: isotropic phase. The figure between two symbols indicates the transition temperature.

Example 1

4-Hydroxy-2,2',3,3',5,5',6,6'-octafluoro-4'-n-alkyloxybiphenyls

4,4'-Dihydroxy-2,2',3,3'5,5',6,6'-octafluorobiphenyl (3 g), (preparation described by Lit.[1]) methylated spirit (10 $cm^3$) and n-pentyl bromide (1.36 g) are gently heated under reflux, with stirring. Potassium hydroxide (0.6 g) in water (10 $cm^3$) is added dropwise to the mixture over 2 h. On completion of the addition, the reaction mixture is stirred and heated under reflux for a further 3 h. The reaction mixture is then cooled, acidified with 4M-aqueous hydrochloric acid, extracted with dichloromethane (3 x 20 $cm^3$), the extract is dried over anhydrous magnesium sulphate, and the solvent removed under reduced pressure. 4-Hydroxy-2,2',3,3',5,5',6,6'octafluoro-4'-n-pentyloxybiphenyl is obtained as a clear oil, b.p. 163-164 °C/0.04 torr.

The following compounds are obtained analogously:
4-hydroxy-2,2',3,3',5,5',6,6'-octafluoro-4'-ethoxybiphenyl
4-hydroxy-2,2',3,3',5,5',6,6'-octafluoro-4'-n-propyloxybiphenyl
4-hydroxy-2,2',3,3',5,5',6,6'-octafluoro-4'-n-butyloxybiphenyl
4-hydroxy-2,2',3,3',5,5',6,6'-octafluoro-4'-n-hexyloxybiphenyl

1) Reference I.L. Knunyants and G.G. Yakobson, Synthesis of Fluoroorganic Compounds, Springer Verlag, Berlin, Heidelberg, New York, Tokyo, 1985, p. 172

4-hydroxy-2,2',3,3',5,5',6,6'-octafluoro-4'-n-heptyloxybiphenyl
4-hydroxy-2,2',3,3',5,5',6,6'-octafluoro-4'-n-octyloxybiphenyl
4-hydroxy-2,2',3,3',5,5',6,6'-octafluoro-4'-n-nonyloxybiphenyl
4-hydroxy-2,2',3,3',5,5',6,6'-octafluoro-4'-n-decyloxybiphenyl

Example 2

2,2',3,3',5,5',6,6'-Octafluoro-4'-alkyloxybiphenyl-4-yl trans-4-n-alkylcyclohexane-1-carboxylates

Trans-4-n-pentylcyclohexane-1-carboxylic acid (0.7 g), and 4-hydroxy-2,2',3,3',5,5',6,6'-octafluoro-4'-n-pentyloxybiphenyl (1.35 g) are added with stirring to dry methylene chloride (20 $cm^3$) contained in a 50 $cm^3$ round-bottomed flask equipped with a calcium chloride guard tube. Trifluoroacetic anhydride (0.95 g) is added and the reaction mixture stirred at room temperature for 15 h. The solvent is removed under reduced pressure and the residue recrystallised from ethanol affording 2,2',3,3',5,5',6,6'-octafluoro-4'-n-pentyloxybiphenyl-4-yl trans-4-n-pentylcyclohexane-1-carboxylate, 1.25 g, as a white crystalline solid, Cr 41.5 °C N 112 °C I.

The following are obtained analogously:
2,2'-3,3',5,5',6,6'-octafluoro-4'-n-pentyloxybiphenyl-4-yl trans-4-ethylcyclohexane-1-carboxylate
2,6'-3,3',5,5',6,6'-octafluoro-4'-n-pentyloxybiphenyl-4-yl trans-4-n-propylcyclohexane-1-carboxylate Cr 58.5 ° N 105.3 ° I
2,2'-3,3',5,5',6,6'-octafluoro-4'-n-pentyloxybiphenyl-4-yl trans-4-n-butylcyclohexane-1-carboxylate
2,2'-3,3',5,5',6,6'-octafluoro-4'-n-pentyloxybiphenyl-4-yl trans-4-n-hexylcyclohexane-1-carboxylate
2,2'-3,3',5,5',6,6'-octafluoro-4'-n-pentyloxybiphenyl-4-yl trans-4-n-heptylcyclohexane-1-carboxylate Cr. 59.5 °, N 102 ° I
2,2'-3,3',5,5',6,6'-octafluoro-4'-n-pentyloxybiphenyl-4-yl trans-4-n-octylcyclohexane-1-carboxylate
2,2'-3,3',5,5',6,6'-octafluoro-4'-n-pentyloxybiphenyl-4-yl trans-4-n-nonylcyclohexane-1-carboxylate
2,2'-3,3',5,5',6,6'-octafluoro-4'-n-pentyloxybiphenyl-4-yl trans-4-n-decylcyclohexane-1-carboxylate
2,2'-3,3',5,5',6,6'-octafluoro-4'-ethoxybiphenyl-4-yl trans-4-ethylcyclohexane-1-carboxylate
2,2'-3,3',5,5',6,6'-octafluoro-4'-ethoxybiphenyl-4-yl trans-4-n-butylcyclohexane-1-carboxylate
2,2'-3,3',5,5',6,6'-octafluoro-4'-ethoxybiphenyl-4-yl trans-4-n-pentylcyclohexane-1-carboxylate
2,2'-3,3',5,5',6,6'-octafluoro-4'-ethoxybiphenyl-4-yl trans-4-n-hexylcyclohexane-1-carboxylate
2,2'-3,3',5,5',6,6'-octafluoro-4'-ethoxybiphenyl-4-yl trans-4-n-heptylcyclohexane-1-carboxylate
2,2'-3,3',5,5',6,6'-octafluoro-4'-ethoxybiphenyl-4-yl trans-4-n-octylcyclohexane-1-carboxylate
2,2'-3,3',5,5',6,6'-octafluoro-4'-ethoxybiphenyl-4-yl trans-4-n-nonylcyclohexane-1-carboxylate
2,2'-3,3',5,5',6,6'-octafluoro-4'-ethoxybiphenyl-4-yl trans-4-n-decylcyclohexane-1-carboxylate
2,2'-3,3',5,5',6,6'-octafluoro-4'-n-octyloxybiphenyl-4-yl trans-4-ethylcyclohexane-1-carboxylate
2,2'-3,3',5,5',6,6'-octafluoro-4'-n-octyloxybiphenyl-4-yl trans-4-n-propylcyclohexane-1-carboxylate
2,2'-3,3',5,5',6,6'-octafluoro-4'-n-octyloxybiphenyl-4-yl trans-4-n-butylcyclohexane-1-carboxylate
2,2'-3,3',5,5',6,6'-octafluoro-4'-n-octyloxybiphenyl-4-yl trans-4-n-pentylcyclohexane-1-carboxylate
2,2'-3,3',5,5',6,6'-octafluoro-4'-n-octyloxybiphenyl-4-yl trans-4-n-hexylcyclohexane-1-carboxylate Cr. 48.5 ° N 94.3° I
2,2'-3,3',5,5',6,6'-octafluoro-4'-n-octyloxybiphenyl-4-yl trans-4-n-heptylcyclohexane-1-carboxylate
2,2'-3,3',5,5',6,6'-octafluoro-4'-n-octyloxybiphenyl-4-yl trans-4-n-octylcyclohexane-1-carboxylate
2,2'-3,3',5,5',6,6'-octafluoro-4'-n-octyloxybiphenyl-4-yl trans-4-n-nonylcyclohexane-1-carboxylate
2,2'-3,3',5,5',6,6'-octafluoro-4'-n-octyloxybiphenyl-4-yl trans-4-n-decylcyclohexane-1-carboxylate

Example 3

4-n-Alkyl-2,2',3,3',4',5,5',6,6'-nonafluorobiphenyls

A vigorously stirred solution of decafluorobiphenyl (20 g) in dry ether (280 $cm^3$) is cooled to -10 °C. In an atmosphere of dry nitrogen 1.6 M n-butyllithium (40 $cm^3$) is added at such a rate that the temperature does not rise above -5 °C. After completion of the addition, the reaction mixture is maintained at -10 °C for 1 h and the temperature is then allowed to rise. When room temperature is reached water (200 $cm^3$) is added, at first dropwise to destroy any unreacted n-butyllithium. The ether layer is separated and the aqueous layer extracted with ether (3 x 120 $cm^3$). The ether solutions are combined, washed with water (200 $cm^3$), dried over anhydrous magnesium sulphate, and the solvent removed under reduced pressure. When the residual oil is purified by short-path distillation 4-n-butyl-2,2',3,3',4',5,5', 6,6'-nonafluorobiphenyl is

obtained as a clear oil, 12.8 g, b.p. 90-95 °C/0.01 torr.

The following compounds are obtained analogously
4-methyl-2,2',3,3',4',5,5',6,6'-nonafluorobiphenyl
4-ethyl-2,2',3,3',4',5,5',6,6'-nonafluorobiphenyl
4-n-propyl-2,2',3,3',4',5,5',6,6'-nonafluorobiphenyl
4-(1-methylpropyl)-2,2',3,3',4',5,5',6,6'-nonafluorobiphenyl
4-n-pentyl-2,2',3,3',4',5,5',6,6'-nonafluorobiphenyl
4-n-hexyl-2,2',3,3',4',5,5',6,6'-nonafluorobiphenyl
4-n-heptyl-2,2',3,3',4',5,5',6,6'-nonafluorobiphenyl
4-n-octyl-2,2',3,3',4',5,5',6,6'-nonafluorobiphenyl

Example 4

4-n-Alkyl-4'-hydroxy-2,2',3,3',4',5,5',6,6'-octafluorobiphenyls

4-n-Butyl-2,2',3,3',4',5,5',6,6'-nonafluorobiphenyl (12 g), potassium hydroxide (18 g) and tert-butyl alcohol (240 $cm^3$) are placed in a 500 $cm^3$ round-bottomed flask equipped with a reflux condenser. The mixture is heated under reflux for 1 h, then water (200 $cm^3$) is added and an aqueous solution of tert-butyl alcohol distilled off until the temperature at the still-head reaches 99-100 °C. The reaction mixture is cooled and acidified with 4M-aqueous hydrochloric acid and extracted with ether (3 x 150 $cm^3$).

The combined extracts are dried over anhydrous magnesium sulphate and the solvent removed under reduced pressure. The crude phenol is purified by flash chromatography on silica gel eluted with 5:1 light petroleum (b.p. 60-80 °C): ethyl acetate. The solvents are removed under reduced pressure whereafter short-path distillation, of the product affords the pure 4-n-butyl-4'-hydroxy-2,2',3,3',5,5', 6,6'-octafluorobiphenyl as a clear oil, 4.0 g, b.p. 190-192/0.1 torr.

The following compounds are obtained analogously:
4-methyl-4'-hydroxy-2,2',3,3',5,5',6,6'-octafluorobiphenyl
4-ethyl-4'-hydroxy-2,2',3,3',5,5',6,6'-octafluorobiphenyl
4-n-propyl-4'-hydroxy-2,2',3,3',5,5',6,6'-octafluorobiphenyl
4-(1-methylpropyl)-4'-hydroxy-2,2',3,3',5,5',6,6'-octafluorobiphenyl
4-n-pentyl-4'-hydroxy-2,2',3,3',5,5',6,6'-octafluorobiphenyl
4-n-hexyl-4'-hydroxy-2,2',3,3',5,5',6,6'-octafluorobiphenyl
4-n-heptyl-4'-hydroxy-2,2',3,3',5,5',6,6'-octafluorobiphenyl
4-n-octyl-4'-hydroxy-2,2',3,3',5,5',6,6'-octafluorobiphenyl

Example 5

2,2',3,3',5,5',6,6'-Octafluoro-4'-n-alkylbiphenyl-4-yl trans-4-n-alkylcyclohexane-1-carboxylates

2,2',3,3',5,5',6,6'-Octafluoro-4'-n-butylbiphenyl-4-yl trans-4-n-pentylcyclohexane-1-carboxylate is prepared and purified in an analogous manner to that described for the esterification of trans-4-n-pentylcyclohexane-1-carboxylic acid with 4-hydroxy-2,2',3,3',5,5',6,6'-octafluoro-4'-n-pentyloxybiphenylin the presence of trifluoroacetic anhydride (cp. example 3) in this case with 4-n-butyl-4'-hydroxy-2,2',3,3',5,5',6,6'-octafluorobiphenyl as starting material. The ester is obtained as a white crystalline solid, Cr 63 °, N 88.5 ° I.

The following compounds are obtained analogously:
2,2',3,3',5,5',6,6'-octafluoro-4'-n-butylbiphenyl-4-yl trans-4-ethylcyclohexane-1-carboxylate
2,2',3,3',5,5',6,6'-octafluoro-4'-n-butylbiphenyl-4-yl trans-4-n-propylcyclohexane-1-carboxylate
Cr 77.5° N 93° I
2,2',3,3',5,5',6,6'-octafluoro-4'-n-butylbiphenyl-4-yl trans-4-n-butylcyclohexane-1-carboxylate
2,2',3,3',5,5',6,6'-octafluoro-4'-n-butylbiphenyl-4-yl trans-4-n-hexylcyclohexane-1-carboxylate
2,2',3,3',5,5',6,6'-octafluoro-4'-n-butylbiphenyl-4-yl trans-4-n-heptylcyclohexane-1-carboxylate
Cr 64 ° N 82.5° I
2,2',3,3',5,5',6,6'-octafluoro-4'-n-butylbiphenyl-4-yl trans-4-n-octylcyclohexane-1-carboxylate
2,2',3,3',5,5',6,6'-octafluoro-4'-n-butylbiphenyl-4-yl trans-4-n-nonylcyclohexane-1-carboxylate
2,2',3,3',5,5',6,6'-octafluoro-4'-n-butylbiphenyl-4-yl trans-4-n-decylcyclohexane-1-carboxylate
2,2',3,3',5,5',6,6'-octafluoro-4'-ethylbiphenyl-4-yl trans-4-ethylcyclohexane-1-carboxylate
2,2',3,3',5,5',6,6'-octafluoro-4'-ethylbiphenyl-4-yl trans-4-n-propylcyclohexane-1-carboxylate

2,2',3,3',5,5',6,6'-octafluoro-4'-ethylbiphenyl-4-yl trans-4-n-butylcyclohexane-1-carboxylate
2,2',3,3',5,5',6,6'-octafluoro-4'-ethylbiphenyl-4-yl trans-4-n-pentylcyclohexane-1-carboxylate
2,2',3,3',5,5',6,6'-octafluoro-4'-ethylbiphenyl-4-yl trans-4-n-hexylcyclohexane-1-carboxylate
2,2',3,3',5,5',6,6'-octafluoro-4'-ethylbiphenyl-4-yl trans-4-n-heptylcyclohexane-1-carboxylate
2,2',3,3',5,5',6,6'-octafluoro-4'-ethylbiphenyl-4-yl trans-4-n-octylcyclohexane-1-carboxylate
2,2',3,3',5,5',6,6'-octafluoro-4'-ethylbiphenyl-4-yl trans-4-n-nonylcyclohexane-1-carboxylate
2,2',3,3',5,5',6,6'-octafluoro-4'-ethylbiphenyl-4-yl trans-4-n-decylcyclohexane-1-carboxylate
2,2',3,3',5,5',6,6'-octafluoro-4'-n-octylbiphenyl-4-yl trans-4-ethylcyclohexane-1-carboxylate
2,2',3,3',5,5',6,6'-octafluoro-4'-n-octylbiphenyl-4-yl trans-4-n-propylcyclohexane-1-carboxylate
2,2',3,3',5,5',6,6'-octafluoro-4'-n-octylbiphenyl-4-yl trans-4-n-butylcyclohexane-1-carboxylate
2,2',3,3',5,5',6,6'-octafluoro-4'-n-octylbiphenyl-4-yl trans-4-n-pentylcyclohexane-1-carboxylate
2,2',3,3',5,5',6,6'-octafluoro-4'-n-octylbiphenyl-4-yl trans-4-n-hexylcyclohexane-1-carboxylate
2,2',3,3',5,5',6,6'-octafluoro-4'-n-octylbiphenyl-4-yl trans-4-n-heptylcyclohexane-1-carboxylate
2,2',3,3',5,5',6,6'-octafluoro-4'-n-octylbiphenyl-4-yl trans-4-n-octylcyclohexane-1-carboxylate
2,2',3,3',5,5',6,6'-octafluoro-4'-n-octylbiphenyl-4-yl trans-4-n-nonylcyclohexane-1-carboxylate
2,2',3,3',5,5',6,6'-octafluoro-4'-n-octylbiphenyl-4-yl trans-4-n-decylcyclohexane-1-carboxylate

Example 6

A mixture is formulated containing
40 % of 2,2',3,3',5,5',6,6'-octafluoro-4'-n-pentyloxybiphenyl-4-yl trans 4-n-pentylcyclohexane-1-carboxylate
30 % of 2,2',3,3',5,5',6,6'-octafluoro-4'-n-pentyloxybiphenyl-4-yl trans-4-n-propylcyclohexane-1-carboxylate
30 % of 2,2',3,3',5,5',6,6'-octafluoro-4'-n-pentyloxybiphenyl-4-yl trans-4-n-heptylcyclohexane-1-carboxylate
The nematic to isotropic transition temperature of this mixture is measured and found to be 102 °C. Further the physical properties of the mixture are measured at 20 °C and found to be
Dielectric anisotropy -0.5
Refractive indices at 589 nm
$n_e$ = 1.5723
$n_o$ = 1.4499
$\Delta n$ = 0.1224

Example 7

A mixture is formulated containing
36 % of 2,2',3,3',5,5',6,6'-octafluoro-4'-n-pentyloxybiphenyl-4-yl trans-4-n-pentylcyclohexane-1-carboxylate
27 % of 2,2',3,3',5,5',6,6'-octafluoro-4'-n-pentyloxybiphenyl-4-yl trans-4-n-propylcyclohexane-1-carboxylate
26 % of 2,2',3,3',5,5',6,6'-octafluoro-4'-n-pentyloxybiphenyl-4-yl trans-4-n-heptylcyclohexane-1-carboxylate
10 % of trans-4-n-propylcyclohexyl-4-benzonitrile
The refractive indices of this mixutre at 20 °C and 577 nm are measured and found to be
$n_e$ = 1.5600
$n_o$ = 1.4581
$\Delta n$ = 0.1019

Example 8

A mixture is formulated containing
33.3 % of 2,2',3,3',5,5',6,6'-octafluoro-4'-n-butylbiphenyl-4-yl trans-4-n-propylcyclohexane-1-carboxylate,
33.3 % of 2,2',3,3',5,5',6,6'-octafluoro-4'-n-butylbiphenyl-4-yl trans-4-n-pentylcyclohexane-1-carboxylate,
33.4 % of 2,2',3,3',5,5',6,6'-octafluoro-4'-n-butylbiphenyl-4-yl trans-4-n-heptylcyclohexane-1-carboxylate
The mixture forms a nematic liquid crystal phase which crystallises slowly on standing on room temperature. The physical properties of the mixture are measured and found to be:
Cr. 44.0 ° N 83 ° I
$\Delta\epsilon$ +0.05
$n_o$ at 20 °C and 589.6 nm = 1.4515

Example 9

An electro-optic display cell constructed of 2 parallel sheets of glass carrying conduction indium tin oxide electrodes on the inner surfaces is filled with the mixture described in example 7. The glass surfaces are further treated with a rubber polymer aligning layer to from a twisted nematic structure within the display cell. The cell is placed between crossed sheets of polarising film and the electro-optic characteristics of the display are evaluated at 20 °C by measuring the percentage transmission of light under different applied voltages.

The following results are found

| | |
|---|---|
| $V_{90,0,20}$ | 3.24 volts |
| $V_{10,0,20}$ | 4.57 volts |
| $V_{50,10,20}$ | 3.38 volts |
| $V_{90,45,20}$ | 2.55 volts |
| $M_{20}$ | 1.77 |
| $M'_{20}$ | 1.50 |

The significance of the voltage above is described by the three subscripts appended to each voltage, in which the first subscript describes the percentage transmission of light through the electro-optic cell when viewed between crossed polarising film, the second subscript gives the angle in degrees away from normal incidence in the quadrant of the cell having the lowest threshold voltage at which the measurement is made, and the third subscript gives the temperature in degrees centigrade at which the measurement is made. The figures $M_{20}$ and $M'_{20}$ defined as below are figures of merit relating to the variation of contrast in the cell under different conditions of angular view. By virtue of the near ideal match of the optical thickness of the cell to the ideal value a good viewing angle characteristic can be obtained.

$M_{20} = V_{10,0,20}/V_{90,45,20}$
$M'_{20} = V_{50,10,20}/V_{90,45,20}$

## Claims

**1.** A liquid crystalline mixture with at least two liquid crystalline compounds characterized by the fact that at least one compound is a liquid crystalline compound of formula I

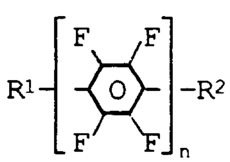

I

wherein n is 2, 3 or 4 and $R^1$ and $R^2$ each signify a group of formula

$R\text{-}(A^1\text{-}Z^1)_p\text{-}(A^2\text{-}Z^2)_q\text{-}$

in which

R is F, OH, Cl, Br, CN, NCO, NCS, $NO_2$, NC or alkyl or perfluoroalkyl wherein alkyl each has 1-15 C atoms, wherein one or two non-adjacent $CH_2$ or $CF_2$ groups, respectively, may be replaced by -O-, -CO-, -O-CO-, -CO-O-, -O-CO-O-, -C≡C- and/or -CH = CH-,

$A^1$ and $A^2$ independently are an unsubstituted or mono- or polysubstituted 1,4-cyclohexylene group, wherein one or two non-adjacent $CH_2$ groups may be replaced by -O- and/or -S-, or denote a 1,4-bicyclo[2,2,2]octylene group, or a 1,4-phenylene group which is unsubstituted or substituted by one or up to four F and/or one or two Cl atoms and/or $CH_3$ groups and/or CN groups, wherein one or two CH groups may be replaced by N,

$Z^1$ denotes -CO-O-, -O-CO-, $-CH_2CH_2-$, $-CHCN\text{-}CH_2-$, $-CH_2\text{-}CHCN-$, -CH = CH-, -C≡C-, $-OCH_2-$, $-CH_2O-$, -N = H-, -NO = N-, -N = NO- or a single bond,

| | |
|---|---|
| $Z^2$ | denotes -CO-O-, -O-CO-, $-CH_2CH_2-$, $-CHCN-CH_2-$, $-CH_2-CHCN-$, -CH=CH-, -C≡C-, $-OCH_2-$, $-CH_2O-$, -N=N-, -NO=N-, -N=NO-, $-(CH_2)_k-CO-O-$ or a single bond, |
| k | is 1 to 6, |
| p | is 0, 1 or 2, and |
| q | is 0 or 1, |

with the proviso that in the case n = 2

(a) the sum of p and q is 1, 2 or 3 and/or

(b) the sum of p and q is 0 and at least one of the rests R is alkyl or perfluoralkyl wherein alkyl hat 1-15 C atoms, wherein one or two non-adjacent $CH_2$ or $CF_2$ groups, respectively, may be replaced by -O-, -CO-, -O-CO-, -CO-O-, -O-CO-O-, -C≡C- and/or -CH=CH-.

**2.** A means of reducing the refractive indices of a liquid crystalline mixture by adding one or more compounds of formula I.

**3.** An electro-optic display device containing a liquid crystalline mixture according to claim 1.

**4.** An optical waveguide switching device containing a liquid crystalline mixture according to claim 1.

**5.** A liquid crystalline compound of formula I

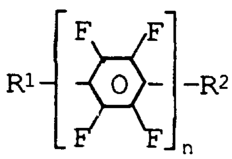

I

wherein n is 2 and $R^1$ and $R^2$ each signify a group of formula

$R-(A^1-Z^1)_p-(A^2-Z^2)_q-$

in which

| | |
|---|---|
| R | is F, OH, Cl, Br, CN, NCO, NCS, $NO_2$, NC or alkyl or perfluoroalkyl wherein alkyl each has 1-15 C atoms, wherein one or two non-adjacent $CH_2$ or $CF_2$ groups, respectively, may be replaced by -O-, -CO-, -O-CO-, -CO-O-, -O-CO-O-, -C≡C- and/or -CH=CH-, |
| $A^1$ and $A^2$ | independently are an unsubstituted or mono- or polysubstituted 1,4-cyclohexylene group, wherein one or two non-adjacent $CH_2$ groups may be replaced by -O- and/or -S-, or denote a 1,4-bicyclo[2,2,2]octylene group, or a 1,4-phenylene group, |
| $Z^1$ | denotes -CO-O-, -O-CO-, $-CH_2CH_2-$, $-CHCN-CH_2-$, $-CH_2-CHCN-$, -CH=CH-, -C≡C-, $-OCH_2-$, $-CH_2O-$, -N=N-, -NO=N-, -N=NO- or a single bond, |
| $Z^2$ | denotes -CO-O-, -O-CO-, $-CH_2CH_2-$, $-CHCN-CH_2-$, $-CH_2-CHCN-$, -CH=CH-, -C≡C-, $-OCH_2-$, $-CH_2O-$, -N=N-, -NO=N-, -N=NO-, $-(CH_2)_k-CO-O-$ or a single bond, |
| k | is 1 to 6, |
| p | is 0, 1 or 2, and |
| q | is 0 or 1, |

with the proviso that

the sum of p and q is 1, 2 or 3

**6.** A compound of part formula Ib

$R-PheF_4-PheF_4-Z^2-A^2-R$ Ib

wherein R, $Z^2$, $A^2$ have the meaning given in claim 5
and
$PheF_4$ is a tetrafluoro-1,4-phenylene group.

**7.** A compound according to claim 6 selected from the group III:
III: alkyl-$PheF_4$-$PheF_4$-A-alkyl
alkyl-$PheF_4$-$PheF_4$-A-alkoxy
alkoxy-$PheF_4$-$PheF_4$-A-alkoxy
alkoxy-$PheF_4$-$PheF_4$-A-alkyl
F-$PheF_4$-$PheF_4$-A-alkyl
F-$PheF_4$-$PheF_4$-A-alkoxy
wherein
alkyl and alkoxy denote alkyl or alkoxy radicals with 2 to 7 C atoms,
$PheF_4$ is a tetrafluoro-1,4-phenylene group
A is a 1,4-phenylene group or a 1,4-cyclohexylene group.

**8.** A compound according to claim 6 selected from the group IV:
IV: alkyl-$PheF_4$-$PheF_4$-CO-O-A-alkyl
alkyl-$PheF_4$-$PheF_4$-CO-O-A-alkoxy
alkoxy-$PheF_4$-$PheF_4$-CO-O-A-alkoxy
alkoxy-$PheF_4$-$PheF_4$-CO-O-A-alkyl
F-$PheF_4$-$PheF_4$-CO-O-A-alkyl
F-$PheF_4$-$PheF_4$-CO-O-A-alkoxy
wherein alkyl, alkoxy, $PheF_4$ and A have the meaning given in claim 7.

**9.** A compound according to claim 6 selected form the group V:
V: alkyl-$PheF_4$-$PheF_4$-O-CO-A-alkyl
alkyl-$PheF_4$-$PheF_4$-O-CO-A-alkoxy
alkoxy-$PheF_4$-$PheF_4$-O-CO-A-alkoxy
alkoxy-$PheF_4$-$PheF_4$-O-CO-A-alkyl
F-$PheF_4$-$PheF_4$-O-CO-A-alkyl
F-$PheF_4$-$PheF_4$-O-CO-A-alkoxy
F-$PheF_4$-$PheF_4$-O-$CH_2$-A-alkyl
F-$PheF_4$-$PheF_4$-O-$CH_2$-A-alkoxy
alkoxy-$PheF_4$-$PheF_4$-O-$CH_2$-A-alkyl
alkoxy-$PheF_4$-$PheF_4$-O-$CH_2$-A-alkoxy
alkyl-$PheF_4$-$PheF_4$-O-$CH_2$-A-alkyl
alkyl-$PheF_4$-$PheF_4$-O-$CH_2$-A-alkoxy
wherein alkyl, alkoxy, $PheF_4$ and A have the meaning given in claim 7.

**Patentansprüche**

**1.** Flüssigkristalline Mischung aus mindestens zwei flüssigkristallinen Verbindungen, dadurch gekennzeichnet, daß mindestens eine Verbindung eine flüssigkristalline Verbindung der Formel I ist,

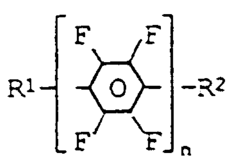 . I

worin n 2, 3 oder 4 bedeutet und $R^1$ und $R^2$ jeweils eine Gruppe der Formel

$$R\text{-}(A^1\text{-}Z^1)_p\text{-}(A^2\text{-}Z^2)_q\text{-}$$

darstellen,
worin
R F, OH, Cl, Br, CN, NCO, NCS, $NO_2$, NC oder Alkyl bzw. Perfluoralkyl, wobei Alkyl jeweils 1-15 C-Atome aufweist, worin eine oder zwei nicht benachbarte $CH_2$-oder $CF_2$-Gruppen jeweils durch -O-, -CO-, -O-CO-, -CO-O-, O-CO-O-, -C≡C- und/oder -CH=CH- ersetzt sein können,

| | |
|---|---|
| $A^1$ und $A^2$ | unabhängig eine nicht substituierte oder einfach oder mehrfach substituierte 1,4-Cyclohexylengruppe, worin eine oder zwei nicht benachbarte $CH_2$-Gruppen durch -O- und/oder -S- ersetzt sein können, oder eine 1,4-Bicyclo[2.2.2]-octylengruppe oder eine gegebenenfalls durch ein oder bis zu vier F- und/oder in oder zwei Cl-Atome und/oder $CH_3$-Gruppen und/oder CN-Gruppen substituierte 1,4-Phenylengruppe, worin eine oder zwei CH-Gruppen durch N ersetzt sein können, |
| $Z^1$ | -CO-O-, -O-CO-, $-CH_2CH_2-$, $-CHCN-CH_2-$, $-CH_2-CHCN-$, -CH = CH-, -C≡C-, $-OCH_2-$, $-CH_2O-$, -N = N-, -NO = N-, -N = NO-oder eine einfache Bindung, |
| $Z^2$ | -CO-O-, -O-CO-, $-CH_2CH_2-$, $-CHCN-CH_2-$, $-CH_2-CHCN-$, -CH = CH-, -C≡C-, $-OCH_2-$, $-CH_2O-$, -N = N-, -NO = N-, -N = NO-, $-(CH_2)_k$-CO-O- oder eine einfache Bindung, |
| k | 1 bis 6, |
| p | 0, 1 oder 2 sowie |
| q | 0 oder 1 bedeuten, |

mit der Maßgabe, daß im Falle von n = 2

(a) die Summe aus p und q 1, 2 oder 3 ist und/oder

(b) die Summe aus p und q 0 ist und mindestens einer der Reste R Alkyl oder Perfluoralkyl bedeutet, wobei Alkyl 1-15 C-Atome aufweist, worin ein oder zwei nicht benachbarte $CH_2$- oder $CF_2$-Gruppen jeweils durch -O-, -CO-, -O-CO-, -CO-O-, -O-CO-O-, -C≡C- und/oder -CH = CH- ersetzt sein können.

**2.** Mittel zur Erniedrigung der Brechungsindices einer flüssigkristallinen Mischung durch Zugabe einer oder mehrerer Verbindungen der Formel I.

**3.** Elektrooptische Anzeigevorrichtung, enthaltend eine flüssigkristalline Mischung nach Anspruch 1.

**4.** Optische Wellenleiterschaltvorrichtung, enthaltend eine flüssigkristalline Mischung nach Anspruch 1.

**5.** Flüssigkristalline Verbindung der Formel I

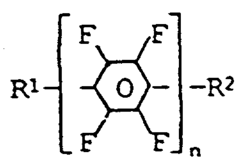

I

worin n 2 bedeutet und $R^1$ und $R^2$ jeweils eine Gruppe der Formel

$R-(A^1-Z^1)_p-(A^2-Z^2)_q-$

darstellen,
worin

| | |
|---|---|
| R | F, OH, Cl, Br, CN, NCO, NCS, $NO_2$, NC oder Alkyl bzw. Perfluoralkyl, wobei Alkyl jeweils 1-15 C-Atome aufweist, worin eine oder zwei nicht benachbarte $CH_2$- oder $CF_2$-Gruppen jeweils durch -O-, -CO-, -O-CO-, -CO-O-, -O-CO-O-, -C≡C- und/oder -CH = CH- ersetzt sein können, |
| $A^1$ und $A^2$ | unabhängig eine nicht substituierte oder einfach oder mehrfach substituierte 1,4-Cyclohexylengruppe, worin eine oder zwei nicht benachbarte $CH_2$-Gruppen durch -O- und/oder -S- ersetzt sein können, oder eine 1,4-Bicyclo[2.2.2]-octylengruppe oder eine 1,4-Phenylengruppe, |
| $Z^1$ | -CO-O-, -O-CO-, $-CH_2CH_2-$, $-CHCN-CH_2-$, $-CH_2-CHCN-$, -CH = CH-, -C≡C-, $-OCH_2-$, $-CH_2O-$, -N = N-, -NO = N-, -N = NO- oder eine einfache Bindung, |
| $Z^2$ | -CO-O-, -O-CO-, $-CH_2CH_2-$, $-CHCN-CH_2-$, $-CH_2-CHCN-$, -CH = CH-, -C≡C-, $-OCH_2-$, $-CH_2O-$, -N = N-, -NO = N-, -N = NO-, $(CH_2)_k$-CO-O- oder eine einfache Bindung, |
| k | 1 bis 6, |
| p | 0, 1 oder 2 sowie |
| q | 0 oder 1 bedeuten, |

mit der Maßgabe, daß die Summe aus p und q 1, 2 oder 3 ist.

**6.** Verbindung der Teilformel Ib

$R\text{-}PheF_4\text{-}PheF_4\text{-}Z^2\text{-}A^2\text{-}R$ Ib

worin R, $Z^2$ und $A^2$ die in Anspruch 5 angegebene Bedeutung besitzen und $PheF_4$ eine Tetrafluor-1,4-phenylengruppe darstellt.

**7.** Verbindung nach Anspruch 6, ausgewählt aus der Gruppe III:

III:
- $Alkyl\text{-}PheF_4\text{-}PheF_4\text{-}A\text{-}Alkyl$
- $Alkyl\text{-}PheF_4\text{-}PheF_4\text{-}A\text{-}Alkoxy$
- $Alkoxy\text{-}PheF_4\text{-}PheF_4\text{-}A\text{-}Alkoxy$
- $Alkoxy\text{-}PheF_4\text{-}PheF_4\text{-}A\text{-}Alkyl$
- $F\text{-}PheF_4\text{-}PheF_4\text{-}A\text{-}Alkyl$
- $F\text{-}PheF_4\text{-}PheF_4\text{-}A\text{-}Alkoxy$

worin
Alkyl und Alkoxy Alkyl- oder Alkoxyreste mit 2 bis 7 C-Atomen,
$PheF_4$ eine Tetrafluor-1,4-phenylengruppe und
A eine 1,4-Phenylengruppe oder eine 1,4-Cyclohexylengruppe bedeuten.

**8.** Verbindung nach Anspruch 6, ausgewählt aus der Gruppe IV:

IV:
- $Alkyl\text{-}PheF_4\text{-}PheF_4\text{-}CO\text{-}O\text{-}A\text{-}Alkyl$
- $Alkyl\text{-}PheF_4\text{-}PheF_4\text{-}CO\text{-}O\text{-}A\text{-}Alkoxy$
- $Alkoxy\text{-}PheF_4\text{-}PheF_4\text{-}CO\text{-}O\text{-}A\text{-}Alkoxy$
- $Alkoxy\text{-}PheF_4\text{-}PheF_4\text{-}CO\text{-}O\text{-}A\text{-}Alkyl$
- $F\text{-}PheF_4\text{-}PheF_4\text{-}CO\text{-}O\text{-}A\text{-}Alkyl$
- $F\text{-}PheF_4\text{-}PheF_4\text{-}CO\text{-}O\text{-}A\text{-}Alkoxy$

worin Alkyl, Alkoxy, $PheF_4$ und A die in Anspruch 7 angegebene Bedeutung besitzen.

**9.** Verbindung nach Anspruch 6, ausgewählt aus der Gruppe V:

V:
- $Alkyl\text{-}PheF_4\text{-}PheF_4\text{-}O\text{-}CO\text{-}A\text{-}Alkyl$
- $Alkyl\text{-}PheF_4\text{-}PheF_4\text{-}O\text{-}CO\text{-}A\text{-}Alkoxy$
- $Alkoxy\text{-}PheF_4\text{-}PheF_4\text{-}O\text{-}CO\text{-}A\text{-}Alkoxy$
- $Alkoxy\text{-}PheF_4\text{-}PheF_4\text{-}O\text{-}CO\text{-}A\text{-}Alkyl$
- $F\text{-}PheF_4\text{-}PheF_4\text{-}O\text{-}CO\text{-}A\text{-}Alkyl$
- $F\text{-}PheF_4\text{-}PheF_4\text{-}O\text{-}CO\text{-}A\text{-}Alkoxy$
- $F\text{-}PheF_4\text{-}PheF_6\text{-}O\text{-}CH_2\text{-}A\text{-}Alkyl$
- $F\text{-}PheF_4\text{-}PheF_4\text{-}O\text{-}CH_2\text{-}A\text{-}Alkoxy$
- $Alkoxy\text{-}PheF_4\text{-}PheF_4\text{-}O\text{-}CH_2\text{-}A\text{-}Alkyl$
- $Alkoxy\text{-}PheF_4\text{-}PheF_4\text{-}O\text{-}CH_2\text{-}A\text{-}Alkoxy$
- $Alkyl\text{-}PheF_4\text{-}PheF_4\text{-}O\text{-}CH_2\text{-}A\text{-}Alkyl$
- $Alkyl\text{-}PheF_4\text{-}PheF_4\text{-}O\text{-}CH_2\text{-}A\text{-}Alkoxy$

worin Alkyl, Alkoxy, $PheF_4$ und A die in Anspruch 7 angegebene Bedeutung besitzen.

## Revendications

**1.** Mélange cristallin liquide comportant au moins deux composés cristallins liquides, caractérisé par le fait qu'au moins un composé est un composé cristallin liquide de formule I

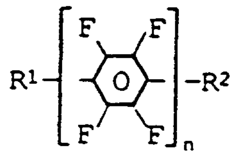

dans laquelle n est égal à 2, 3 ou 4 et $R^1$ et $R^2$ représentent chacun un groupe de formule

$R\text{-}(A^1\text{-}Z^1)_p\text{-}(A^2\text{-}Z^2)_q\text{-}$

dans laquelle

R est F, OH, Cl, Br, CN, NCO, NCS, $NO_2$, NC ou un groupe alkyle ou perfluoroalkyle, dans lequel chaque groupe alkyle possède 1-15 atomes de C, où un ou deux groupes $CH_2$ ou $CF_2$ non adjacents peuvent être respectivement remplacés par -O-, -CO-, -O-CO-, -CO-O-, -O-CO-O-, -C≡C- et/ou -CH = CH-,

$A^1$ et $A^2$ sont chacun indépendamment un groupe 1,4-cyclohexylène non substitué ou mono- ou poly-substitué, dans lequel un ou deux groupes $CH_2$ non adjacents peuvent être remplacés par -O- et/ou -S-, ou désignent un groupe 1,4-bicyclo[2,2,2] octylène, ou un groupe 1,4-phénylène qui est non substitué ou substitué par un ou jusqu'à 4 atomes de F et/ou par un ou deux atomes de Cl et/ou groupes $CH_3$ et/ou groupes CN, où un ou deux groupes CH peuvent être remplacés par N,

$Z^1$ représente -CO-O-, -O-CO-, $-CH_2CH_2-$, $-CHCN-CH_2-$, $-CH_2-CHCN-$, -CH = CH-, -C≡C-, $-OCH_2-$, $-CH_2O-$, -N = N-, -NO = N-, -N = NO- ou une simple liaison,

$Z^2$ représente -CO-O-, -O-CO-, $-CH_2CH_2-$, $-CHCN-CH_2-$, $-CH_2-CHCN-$, -CH = CH-, -C≡C-, $-OCH_2-$, $-CH_2O-$, -N = N-, -NO = N-, -N = NO-, $-(CH_2)_k-CO-O-$ ou une simple liaison,

k a une valeur de 1 à 6,

p est égal à 0, 1 ou 2, et

q est égal à 0 ou 1,

à condition que, dans le cas où n = 2,

(a) la somme de p et q soit égale à 1, 2 ou 3 et/ou

(b) la somme de p et q soit nulle et au moins un des radicaux R soit un groupe alkyle ou perfluoroalkyle, dans lequel le groupe alkyle possède 1-15 atomes de C, où un ou deux groupes $CH_2$ ou $CF_2$ non adjacents peuvent être respectivement remplacés par -O-, -CO-, -O-CO-, -CO-O-, -O-CO-O-, -C≡C- et/ou -CH = CH-.

**2.** Procédé pour abaisser les indices de réfraction d'un mélange cristallin liquide par addition d'un ou plusieurs composés de formule I.

**3.** Dispositif d'affichage électro-optique contenant un mélange cristallin liquide selon la revendication 1.

**4.** Dispositif commutateur de guide d'onde optique contenant un mélange cristallin liquide selon la revendication 1.

**5.** Composé cristallin liquide de formule I

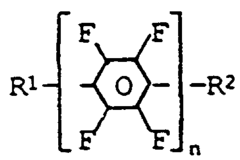

dans laquelle n est égal à 2 et $R^1$ et $R^2$ représentent chacun un groupe de formule

$R-(A^1-Z^1)_p-(A^2-Z^2)_q-$

dans laquelle

R est F, OH, Cl, Br, CN, NCO, NCS, $NO_2$, NC ou un groupe alkyle ou perfluoroalkyle, dans lequel chaque groupe alkyle possède 1-15 atomes de C, où un ou deux groupes $CH_2$ ou $CF_2$ non adjacents peuvent être respectivement remplacés par -O-, -CO-, -O-CO-, -CO-O-, -O-CO-O-, -C≡C- et/ou -CH = CH-,

$A^1$ et $A^2$ sont chacun indépendamment un groupe 1,4-cyclohexylène non substitué ou mono- ou poly-substitué, dans lequel un ou deux groupes $CH_2$ non adjacents peuvent être remplacés par -O- et/ou -S-, ou désignent un groupe 1,4-bicyclo[2,2,2] octylène, ou un groupe 1,4-phénylène,

$Z^1$ représente -CO-O-, -O-CO-, $-CH_2CH_2-$, $-CHCN-CH_2-$, $-CH_2-CHCN-$, -CH = CH-, -C≡C-, $-OCH_2-$, $-CH_2O-$, -N = N-, -NO = N-, -N = NO- ou une simple liaison,

$Z^2$ représente -CO-O-, -O-CO-, $-CH_2CH_2-$, $-CHCN-CH_2-$, $-CH_2-CHCN-$, -CH = CH-, -C≡C-,

-$OCH_2$ -, -$CH_2O$-, -N = N-, -NO = N-, -N = NO-, -$(CH_2)_k$-CO-O- ou une simple liaison,

k a une valeur de 1 à 6,

p est égal à 0, 1 ou 2, et

q est égal à 0 ou 1,

à condition que

la somme de p et q soit égale à 1, 2 ou 3.

**6.** Composé de formule Ib

R-$PheF_4$-$PheF_4$-$Z^2$-$A^2$-R Ib

dans laquelle R, $Z^2$, $A^2$ ont les significations indiquées dans la revendication 5 et $PheF_4$ est un groupe tétrafluoro-1,4-phénylène.

**7.** Composé selon la revendication 6, choisi dans le groupe III :

III : alkyle-$PheF_4$-$PheF_4$-A-alkyle
alkyle-$PheF_4$-$PheF_4$-A-alcoxy
alcoxy-$PheF_4$-$PheF_4$-A-alcoxy
alcoxy-$PheF_4$-$PheF_4$-A-alkyle
F-$PheF_4$-$PheF_4$-A-alkyle
F-$PheF_4$-$PheF_4$-A-alcoxy

dans lequel alkyle et alcoxy désignent des radicaux alkyle ou alcoxy comportant 2 à 7 atomes de C, $PheF_4$ est un groupe tétrafluoro-1,4-phénylène A est un groupe 1,4-phénylène ou un groupe 1,4-cyclohexylène.

**8.** Composé selon la revendication 6, choisi dans le groupe IV :

IV : alkyle-$PheF_4$-$PheF_4$—CO-O-A-alkyle
alkyle-$Phef_4$-$Phef_4$—CO-O-A-alcoxy
alcoxy-$PheF_4$-$PheF_4$—CO-O-A-alcoxy
alcoxy-$PheF_4$-$PheF_4$—CO-O-A-alkyle
F-$PheF_4$-$PheF_4$—CO-O-A-alkyle
F-$PheF_4$-$PheF_4$—CO-O-A-alcoxy

dans lequel alkyle, alcoxy, $PheF_4$ et A ont les significations indiquées dans la revendication 7.

**9.** Composé selon la revendication 6, choisi dans le groupe V :

V : alkyle-$PheF_4$-$PheF_4$—O-CO-A-alkyle
alkyle-$PheF_4$-$PheF_4$—O-CO-A-alcoxy
alcoxy-$PheF_4$-$PheF_4$—O-CO-A-alcoxy
alcoxy-$PheF_4$-$PheF_4$—O-CO-A-alkyle
F-$Phef_4$-$Phef_4$—O-CO-A-alkyle
F-$PheF_4$-$PheF_4$—O-CO-A-alcoxy
F-$PheF_4$-$PheF_4$—O-$CH_2$-A-alkyle
F-$PheF_4$-$PheF_4$—O-$CH_2$-A-alcoxy
alcoxy-$PheF_4$-$PheF_4$—O-$CH_2$-A-alkyle
alcoxy-$PheF_4$-$PheF_4$—O-$CH_2$-A-alcoxy
alkyle-$PheF_4$-$PheF_4$—O-$CH_2$-A-alkyle
alkyle-$PheF_4$-$PheF_4$—O-$CH_2$-A-alcoxy

dans lequel alkyle, alcoxy, $PheF_4$ et A ont les significations indiquées dans la revendication 7.